# EUROPEAN PATENT SPECIFICATION

(11) **EP 2 580 318 B1**
(45) Date of publication and mention of the grant of the patent: **29.08.2018**
(21) Application number: 11793275.6
(22) Date of filing: 10.06.2011
(51) Int. Cl.: C12N 5/04, A01N 25/18, A01N 31/02, A01N 31/16

(54) **ATTRACTANT COMPOSITIONS FOR WEEVILS OF THE GENUS OTIORHYNCHUS AND USES THEREOF**
LOCKSTOFF-ZUSAMMENSETZUNGEN FÜR RÜSSELKÄFER DER GATTUNG DICKMAULRÜSSLER UND VERWENDUNGEN DAVON
COMPOSITIONS D'ATTRACTIF POUR CHARANÇONS DU GENRE OTIORHYNCHUS ET LEURS UTILISATIONS

(30) Priority: 10.06.2010 US 353568 P; 10.06.2011 US 201113157944
(43) Date of publication of application: 17.04.2013
(73) Proprietor: The United States of America as represented by The Secretary of Agriculture, Washington, DC 20250 (US); Stichting Wageningen Research, 6701 BH Wageningen (NL)
(72) Inventor: BRUCK, Denny J., Corvallis, OR 97330 (US); VAN TOL, Robert W.H.M., 6700 AH Wageningen (NL); GRIEPINK, Frans, C., 6700 AH Wageningen (NL)
(74) Representative: Touroude, Magali Linda
(86) International application number: PCT/US2011/040054
(87) International publication number: WO 2011/156762

(56) References cited:
- US-A- 4 819 371
- US-A1- 2009 074 809
- US-A1- 2009 082 355
- US-A1- 2009 288 333
- US-B1- 6 183 733
- VAN TOL ET AL: "Host-plant preference and antennal responses of the black vine weevil (Otiorhynchus sulcatus) to plant volatiles", EXPERIMENTAL AND APPLIED ENTOMOLOGY,, vol. 9, 1 January 1998 (1998-01-01), pages 35-40, XP009177417,
- Comp B Comp ET AL: "Semiochemical Tested Ratio of O. sulcatus captured (odor/control) component A component A+B Development of Biologically-Based Strategies for Managing Insect Pests of Horticultural Crops", , 1 October 2009 (2009-10-01), XP55111994, Retrieved from the Internet: URL:http://www.ars.usda.gov/SP2UserFiles/P lace/02060000/FNRI2009/FNRI_2009_ProgressR eports/Bruck 2009 Research Reports-EDITED.pdf [retrieved on 2014-04-03]
- VAN TOL ET AL.: 'Olfactory antennal responses of the vine weevil Otiorhynchus sulcatus to plant volatiles' ENTOMOLOGIA EXPERIMENTALIS ET APPLICATA vol. 102, no. 1, January 2002, pages 49 - 64, XP055070803
- WARTHEN ET AL.: 'Volatile, potential attractants from ripe coffee fruit for female mediterranean fruit fly' JOURNAL OF CHEMICAL ECOLOGY vol. 23, no. 7, 1997, pages 1891 - 1900, XP055070805
- NOWINSZKY L.: 'Nocturnal illumination and night flying insects' APPLIED ECOLOGY AND ENVIRONMENTAL RESEARCH vol. 2, no. 1, 2004, pages 17 - 52, XP055070809

## Description

### FIELD OF THE INVENTION

The invention relates to the use of volatile organic compounds effective for attracting *Otiorhynchus* weevil species (*Otiorhynchus* sp.) and to trap containing said volatile organic compounds to control *Otiorhynchus* weevils.

### BACKGROUND OF THE INVENTION

Vine weevils of the genus *Otiorhynchus* are an important pest of hardy ornamentals, fruit tree and nursery stock worldwide. Though *Otiorhynchus* is of European origin, it has travelled from Europe to other regions of the world via plant material and thus, has become one of the most destructive pests in nursery and small fruit production areas throughout the United States and Canada as well as Europe.

Typically, adult vine weevils feed on plant leaves and deposit eggs in the soil. Once hatched, the larvae, born in the soil, feed on plant roots weakening and sometimes, killing the plants. Accordingly, *Otiorhynchus* weevils are responsible for a considerable amount of economically important damage. Indeed, based on USDA data, $25-$70 million is spent annually in the USA and Canada alone to combat this worldwide horticultural pest.

Unfortunately, combating *Otiorhynchus* weevil attack is problematic because adult weevils are active at night. The nocturnal behavior makes monitoring and timing of control measures difficult because growers frequently are not able to observe the emerging weevils in a timely manner. Thus, oviposition and thus, the next crop of destructive larvae, often starts before effective control measures are taken.

Typically, weevil presence is determined by monitoring feeding damage to plants. The most extensive feeding occurs during the four week preoviposition period and several weeks during oviposition. If adult weevils can be located and either captured or killed before they lay eggs, and initiate a new generation, much damage can be averted.

Unfortunately, localizing affected areas in nurseries is labor intensive, especially since the adults are active at night and hide during the day. Thus, to combat weevil infestation without encountering excessive labor costs, growers typically apply broad spectrum pesticides over the entire area of affected or potentially affected plants for the entire growing season, which typically lasts from June to October. Amongst other issues, the use of broad spectrum pesticides over a large area for a prolonged period, limits the ability of growers to practice less toxic integrated pest management techniques.

Therefore, what is needed in the art are effective means for monitoring and controlling *Otiorhynchus* weevils before oviposition such that damage to nursery stock can be minimized or eliminated.

Fortunately, as will be clear from the following disclosure, the present invention provides for these and other needs.

### SUMMARY OF THE INVENTION

In an exemplary aspect, the invention provides the use of a composition of one or more attractant volatile organic compounds effective for attracting *Otiorhynchus* weevils, the volatile organic compounds having at least 85 % purity selected from the group consisting of (*X*)-Y-pentenol, methyl eugenol, methyl *iso*-eugenol and a combination of said members, wherein *X* is E or Z, and Y is 1 or 2. In one exemplary embodiment, the *Otiorhynchus* weevils are *Otiorhynchus* sulcatus. In another exemplary embodiment of the present invention, the attractant volatile organic compound used is *(Z)*-2-pentenol of at least 85 % purity.. In another exemplary embodiment of the present invention, the one or more attractant volatile organic compounds used are (*Z*)-2-pentenol and methyl eugenol of at least 85 % purity. In still another exemplary embodiment of the present invention, the *Otiorhynchus* weevils are members selected from the group consisting of *Otiorhynchus ovatus* and *Otiorhynchus rugosostriatus,* and the attractant volatile organic compounds used consists of (*Z*)-2-pentenol of at least 85 % purity.

In another exemplary aspect, the invention provides a trap for capturing *Otiorhynchus* weevils, wherein the trap comprises a container that has an interior and an exterior, wherein the container comprises openings in the exterior that are large enough for the weevils to pass through and thereby enter the interior; and whereinthe interior contains a "ruffle" which serves as a hiding place for weevils that enter the trap and wherein the trap is baited with a composition consisting of attractant volatile organic compounds of at least 85 % purity that are members selected from the group consisting of (*X*)-Y-pentenol , methyl eugenol, methyl *iso*-eugenol and a combination of such members, wherein X is E or Z, and Y is 1 or 2. In one exemplary embodiment of the present invention, the trap is placed in a tree or other plant wherein it is desired that weevils are to be trapped/captured/monitored. In another exemplary embodiment, In another exemplary embodiment of the present invention, the trap is baited with a composition consisting of attractant (*Z*)-2-pentenol of at least 85 % purity. In still another exemplary embodiment of the present invention, the trap is baited with a composition consisting of attractant (*Z*)-2-pentenol and methyl eugenol of at least 85 % purity. In still another exemplary embodiment of the present invention,the trap is baited with a composition consisting of attractant volatile organic compounds of at least 85 % purity that are members selected from the group consisting of (*Z*)-2-pentenol, methyl eugenol and a combination of such members. In still another exemplary embodiment of the present invention, the trap further contains a bait laced with synthetic insecticide or pathogens. In still another exemplary embodiment of the present invention,the pathogens are members selected from the group consisting of bacteria, fungi, nematodes and microspora. In still another exemplary embodiment of the present invention, the trap is used in conjunction with light at an intensity of between about 0.1-1.1 lux for the duration of overnight hours.

Other features, objects and advantages of the invention will be apparent from the detailed description which follows.

### BRIEF DESCRIPTION OF THE DRAWINGS

**FIG. 1** Shows different pattern in release of compoundsfrom the headspace of mechanically damaged and weevil-damaged *E.fortunei* 'Dart's Blanket' plants.
**FIG. 2** Show the number of weevils per replicate for each treatment after statistical analysis of the total number of each weevil species caught during the whole season.
**FIG. 3** Illustrates an exemplary "weevil trap".
**FIG. 4** Is a graphical depiction of data obtained from field studies that demonstrate the capture efficacy of several *Otiorhynchus* sp. trap design. *See,* Example 3.

### DETAILED DESCRIPTION OF THE INVENTION

### Definitions

Unless defined otherwise, all technical and scientific terms used herein generally have the same meaning as commonly understood by one of ordinary skill in the art to which this invention belongs.

The term "*Otiorhynchus* weevil", "*Otiorhynchus* weevils", "*Otiorhynchus* sp.", "vine weevil(s)", and/or "weevil(s)" as used herein, refer to weevil species of the genus *Otiorhynchus. "Otiorhynchus* sp." are a major insect pest of agriculture. Exemplary *Otiorhynchus* weevils include, but are not limited to *Otiorhynchus sulcatus* (Black vine weevil), *Otiorhynchus ovatus, Otiorhynchus salicicola, Otiorhynchus rugosostriatus,* etc.

As used herein, the term "control" or "controlling" as in *e.g.,* the phrase: the "control" of *Otiorhynchus* sp., or "controlling" *Otiorhynchus* weevils, or as in the phrase: "controlling" agricultural pests, refers to any means for preventing infection or infestation, reducing the population of already infected areas or organisms, or elimination or reduction in numbers of the population of pests *e.g., Otiorhynchus* sp. or other species whose "control" is desired. Indeed, "controlling" as used herein refers to any indicia of success in prevention, elimination, reduction or amelioration of a pest population or pest problem. In an exemplary embodiment, *Otiorhynchus* weevils are "controlled" by attracting *Otiorhynchus* weevils using volatile organic compounds as disclosed herein and trapping and/or killing the attracted weevils.

The term "reduce" as used herein refers to any indicia of success in the diminishment in size, amount, extent, and/or severity of *Otiorhynchus* weevil infestation. The term "reduce" as used herein also refers to any indicia of success in the diminishment of reproductive capacity (e.g., through killing or trapping etc); diminishment of spread (*e.g.,* rate or extent of spread) e.g., from an un-treated nursery stock to a treated nursery stock; diminished damage to nursery stock or other susceptible plant species caused by *Otiorhynchus* weevils (adults and/or larvae) etc.

As used herein, the term "attracting" refers to the action of causing an insect pest *e.g.,* an *Otiorhynchus* weevil *e.g., Otiorhynchus sulcatus* either directly or indirectly, to move in a direction towards the source of a stimulus e.g. toward a selected attractant VOC as disclosed herein. One of skill in the art will recognize that suitable stimuli may include a large variety of methods including, but not limited to chemical stimulus *e.g*., volatile chemicals such as *e.g.,* those disclosed herein, e.g., (Z)-2-pentenol; pheromones; kairomones; etc. A chemical stimulus can be an individual compound or a composition, including *e.g*., more than one compound *e.g.,* a 1:1 ratio mixture of (*Z*)-2-pentenol and methyl eugenol, that either directly or indirectly, causes the insect to move toward the source of the stimulus.

Thus, the term "attractant" as used herein refers to a stimulus such as e.g., an attractant VOC *e.g.,* (*Z*)-2-pentenol, which causes an *Otiorhynchus* weevil, either directly or indirectly, to move in a direction towards the stimulus. In some exemplary embodiments, an "attractant" takes the form of a "bait composition". Typically, a bait composition comprises at least one attractant VOC as disclosed herein either together with other attractant or non-attractant chemicals or alone in amounts effective to attract *Otiorhynchus* weevils to the bait composition. For *Otiorhynchus* attraction, attractant VOCs are effectively used in a concentration range of from about 1.0% to about 10% (wt:v) for granular baits. Indeed, attractant VOCs disclosed herein are typically 10-100 fold less well sensed by the weevils than pheromones and/or have to compete with natural high background levels of these compounds released by the plants. Thus when attracting Otiorhynchus weevils, exemplary effective amounts typically are about 1%, 2%, 3%, 4%, 5%, 6%, 7%, 8%, 9%, 10% or even more. Thus, a person having of ordinary skill in the art is readily able determine optimal concentration ranges for the attraction of any given attractant VOC.

The term "bait" as used herein, refers to an ingredient or combination of ingredients comprising an attractant.

The term "pheromone" as used herein, refers to a substance or mixture of substances which are secreted and released by an organism for detection and response by another organism of the same species. Pheromones mediate a variety of interactions between organisms. Thus, pheromones are typically classified by the interaction that they most strongly influence *e.g.,* alarm, aggregation or sex pheromone.

As is known in the art, "pheromones" belong to the larger class of chemical compounds referred to as semiochemicals. The term "semiochemical" as used herein refers to chemicals that mediate interactions between organisms. Semiochemicals include allelochemicals and pheromones depending on whether the interactions are interspecific or intraspecific, respectively. As used herein the term "allelochemical" refers to chemical substances that induce a response in the receiver of the signal that is either adaptively favorable to the emitter but not the receiver (allomones), or that is favorable to the receiver but not the emitter (kairomones) or is favorable to both emitter and receiver (synomones). Allelochemicals and pheromones are useful *e.g,* as arrestants, attractants, repellents, deterrents, and/or stimulants.

The term "ratio" as used herein, refers to the relative proportion of at least two compounds with respect to one another.

The term "nursery stock" as used herein, refers broadly to field-grown and/or container-grown hardy perennial and woody plants or biennial trees, shrubs, vines, and plants, evergreens, fruit pits, and other plants or plant parts capable of propagation e.g., vegetative or propagative parts. Other plants considered nursery stock include plants grown for commercial purposes, such as e.g., flowering annuals, strawberry, blueberry, caneberries, etc and vegetables for transplanting.

The terms "isolated," "purified" or "biologically pure" as used herein, refer to a chemical or microorganism that is substantially or essentially free from components that normally accompany it as found in its native state.

In some exemplary embodiments, the term "isolated" is used to describe an isolated chemical compound, e.g., isolated volatile organic compound, e.g., (Z)-2-pentenol. Thus, in some exemplary embodiments, the terms "isolated" or "purified" refer to a chemical species that that is the predominant species present in a preparation. Thus, in some exemplary embodiments, "purity" of an isolated species is determined using analytical chemistry techniques such as *e.g.,* high performance liquid chromatography. A chemical species that is the predominant species present in a preparation is substantially purified. Typically, a "purified" chemical species denotes that a chemical species that is at least about 85% pure, at least about 95% pure, or at least about 99% pure.

The expression "effective amount" or "amount effective for" or any grammatically equivalent expression as used herein, refers to that amount of attractant e.g., a VOC as disclosed herein e.g., (*Z*)-2-pentenol, which is sufficient to attract *Otiorhynchus* weevils, e.g., *Otiorhynchus sulcatus*, relative to a control that does not contain the attractant as disclosed herein. In an exemplary embodiment, an effective amount is that quantity of an attractant VOC or attractant VOC blend that provides a release rate of the attractant VOC(s) that attracts *Otiorhynchus* weevils to the location of a bait at a rate higher than the rate at which weevils are attracted to a nonbaited location. In an exemplary embodiment, an effective amount is provided at a release rate that is about 0.06 ml/day. In another exemplary embodiment, an effective amount is provided at a release rate that is about 0.0024 ml/hour. In another exemplary embodiment, an effective amount is provided at a release rate that is between about 0.02 ml/day to 0.1 ml/day. However, effective release rates are not limited to theses rates and any suitable rate may be effective so long as it is sufficient to attract *Otiorhynchus* weevils to the vicinity or location of a bait comprising the attractant VOC(s) at a rate higher that is than the rate at which weevils are attracted to a nonbaited location.

As used herein, the term "trap" refers to any device into which the volatile organic compounds or blends of compounds *e.g*., (*Z*)-2-pentenol; a 1:1 ratio mixture of *(Z*)-2-pentenol and methyl eugenol, etc as disclosed herein are placed such that *Otiorhynchus* weevils are attracted to the trap and can be monitored, collected and/or killed. In some exemplary embodiments a "trap" prevents the *Otiorhynchus* weevil from escaping once the weevil has come into contact with the trap. However, in other exemplary embodiments, a "trap" effective for controlling *Otiorhynchus* weevils does not prevent the weevil from escaping. Indeed, since *Otiorhynchus* weevils hide during daylight hours, in some exemplary embodiments effective "traps" for *Otiorhynchus* weevils simply provide a daytime hiding place for the weevils. Thus, in an exemplary embodiment, a piece of paper placed on a baited piece of ground is an effective "trap" for *Otiorhynchus* weevils. Weevils hiding in such a "trap" can be collected and/or killed by a person monitoring a nursery or nursery stock for *Otiorhynchus* weevils. Traps can be of various sizes, shapes, colors, and materials. In an exemplary embodiment, traps are designed and manufactured specifically for use as an insect trap. In other exemplary embodiments a trap is a container converted and adapted from other uses such as, for example, a glass Petri dish, a metal coffee can, a cardboard box or any ordinary plastic, paper, metal, wood, fiberglass, composite or ceramic container, etc. Exemplary materials for use in making the traps include, but are not limited to, paper, cardboard, metal, wood, metal alloys, glass, paper, plastic, acrylic, fiberglass, composite, ceramic, etc. Typically, traps have a bottom, sidewalls, and a top. The bottom, sidewalls and top of the trap can be solid, or be perforated. An exemplary perforated sidewall is a screen. In an exemplary embodiment, traps are configured such that insect pests can enter the trap but are unable to escape once inside the trap. In other exemplary embodiments, traps are commercially available *(e.g*., from Suterra Inc.). In still other exemplary embodiments, traps have only a bottom or only a top e.g., a piece of paper placed on a baited piece of ground.

### I. Introduction:

Insects have plagued people throughout history. Intercontinental travel and trade have enabled the importation of *Otiorhynchus* weevils into countries to which they are not indigenous. As a result, numerous species of *Otiorhynchus* weevils now plague the horticultural industry.

Root weevils of the genus *Otiorhynchus*, are flightless parthenogenic and/or sexual weevil species (*see e.g.,* Downes, 1922; Smith, 1932; Suomalainen et al., 1987) that have become a major worldwide pest of the horticultural industry.

Although a number of natural enemies, such as hedgehogs, frogs,predatory beetles, and insect pathogens such as entomopathogenic fungi and nematodes help to maintain *Otiorhynchus* weevils populations at a low level in natural environments, such natural predators and pathogens are less successful or costly and difficult to apply in intensive horticultural systems where persistent chemicals have been relied on to keep populations low and/or when the number of cultured plants is beyond the capacity of the ecosystem.

Adult weevils are nocturnal and oviposition, which produces the next generation of destructive larvae, typically occurs at night. Thus, monitoring and control of the weevils is difficult.

Weevil presence is typically determined by monitoring weevil damage to the plants. To prevent weevil oviposition, growers have available a number of more or less effective measures such as various cultural measures, and chemical and biological agents that can be deployed both above and below ground as necessary (*see e.g.,* Kakouli-Duarte, et al. (1997) Annals of Applied Biology, 131: 11-27; Lola-Lutz. T. and Downes, M. (2007) Biological Control 40(3): 314-319; Moorehouse, E. et al. (1992) Annals of Applied Biology, 121: 431-454; Van Tol, R.W.H.M. and M.J. Raupp (2005) Nursery and tree application (Chapter 9). In: Nematodes as biological control agents (Eds. P.S. Grewal, R-U. Ehlers and D.I. Shapiro-Ilan). CABI Publishing, 167-190; Dolmans, N.G.M. and R.W.H.M. Van Tol (1996). Prospects for chemical control of black vine weevil (Otiorhynchus sulcatus) in nursery stock. Mitteilungen aus der Biologischen Bundesanstalt für Land- und Forstwirtschaft 316, 108-112; Van Tol, R.W.H.M. (1996). A strategy for control of black vine weevil (Otiorhynchus sulcatus) in an Integrated Pest Management programme in nursery stock. Mitteilungen aus der Biologischen Bundesanstalt für Land- und Forstwirtschaft 316, 76-80.). According to Van Tol et al. in "Host-Plant Preference and Antennal Responses of the Black Vine Weevil (Otiorhynchus sulcatus) to Plant Volatiles"(Experimental and Applied Entomology, vol. 9, 1 January 1998, pages 35-40), the black vine weevil (Otiorhynchus sulcatus) shows a distinct preference for certain plant species. Van Tol et al. performed electroantennogram (EAG) tests to screen plant volatiles and demonstrate a highly specific response profile. Though prior art such as Van Tol may use EAG tests to determine such a preference, there remains a need for compositions of volatile organic compounds effective for attracting Otiorhynchus weevil species and to methods for using such volatile organic compounds to control Otiorhynchus weevils.

Unfortunately, as noted above, adults are nocturnal and oviposition typically occurs at night. Thus, a major problem in combating weevil attack is monitoring and timing of control measures. Indeed, due to the night-activity of the adults, growers and gardeners typically do not observe the first emerging weevils in a timely manner and oviposition often starts before effective control measures are taken.

Fortunately however, the instant invention provides, for the first time, compositions of volatile organic chemicals (VOC) effective for attracting *Otiorhynchus* weevils so that they can be effectively monitored and/or controlled despite their nocturnal habits. The compositions and methods utilize a unique combination of VOCs to not only attract *Otiorhynchus* weevils but also to induce them to enter a trap or get in contact with an active ingredient such as a chemical or biological agent so that they can be captured and killed or otherwise controlled.

Thus, as disclosed herein, the effective volatile organic compounds are deployed in the field *e.g.,* nurseries, gardens, etc, for the control of *Otiorhynchus* weevils.

### II. Compounds

### A. General Methods

Methods disclosed herein utilize routine techniques in the field of chemistry and chemical analysis. Basic texts disclosing the general methods of use in this invention include, *e.g.,* GC-MS A Practical User's Guide by Marvin McMaster, Wiley-VCH (1998); Modern Analytical Chemistry, by David T. Harvey, McGraw-Hill Science/Engineering/Math (1999).

### B. Volatile Organic Compounds Effective for Attracting Otiorhynchus weevils

In exemplary embodiments (*E*)-2-hexenol, (*Z*)-2-pentenol, methyl eugenol are used alone in combination to effectively attract *Otiorhynchus* weevils. Typically, volume ratios are 1:1 or 1:1:1 for single, double and triple mixtures of (*E*)-2-hexenol, (Z)-2-pentenol, methyl eugenol.

### III. Methods for Attracting Otiorhynchus weevils

Insect traps are typically used to monitor or directly reduce insect populations. Traps may reduce insect populations directly or may reduce future populations by negatively affecting the reproductive capacity of a present generation of insects e.g., by directly or indirectly preventing oviposition. Thus, one embodiment, the volatile organic compounds disclosed herein, are used to attract and trap *Otiorhynchus* weevils.

Fortunately, it has now been discovered that *Otiorhynchus* weevils of are selectively attracted by a well defined release of attractive chemicals. The ability to attract *Otiorhynchus* weevils before they lay eggs adds greatly to the value of the trapping system because it permits the elimination or reduction of the very destructive larval state.

The addition of supplemental lighting at night alters the catch of *Otiorhynchus* sp. in trap devices. Indeed, light provided at a low intensity level (0.1-1.1 lux) for the duration of the overnight hours demonstrates a synergistic effect of supplemental light with experimental trap B in the overnight hours (see e.g. Example 2 and Table 1 hereinbelow).

### A. Determining an Effective Amount of VOC for Attracting Otiorhynchus weevils

In an exemplary embodiment, effective attractant ability is indicated when capture of *Otiorhynchus* weevils in locations baited with one or more volatile organic compounds as disclosed are higher than in an unbaited location.

Typically, because the attractant VOC(s) disclosed herein are about 10-100 fold less well sensed by the weevils than pheromones and/or have to compete with natural high background levels of these compounds released by the plants effective amounts of VOC(s) for attracting *Otiorhynchus* weevils is typically between about 1% to about 10%. Methods for determining release rates are known in the art (see e.g., R.W.H.M. van Tol et al. (2007) Pest Manag Sci 63: 483-490; Pietrogrande and Basaglia (2007) Trends in Analytical Chemistry 26: 1086-1094; Yosha et al. (2008) J. Agric. Food Chem. 56: 8045-8049).

In an exemplary embodiment, effective release rates are achieved by Placplacing 0.4 ml of pure compounds/mixtures in high volume plastic containers as follows "Plant volatile dispensers were made of 1.5 ml LDPE Pasteur pipettes (Labo Scientific, Ede, the Netherlands). The compounds to be tested were introduced into the pipette, the tip of which was then sealed by heat. Prior to use, the tip of the pipette was cut off at 1 cm above the reservoir portion. The open tip of the dispenser had an internal diameter of 3.5 mm. This type of 'high release' pheromone/kairomone dispenser have bee developed and used by Pherobank® for several years for the attraction of *Phyllopertha horticola* L. (Coleoptera: Scarabaeidae) and has also been successfully tested for attraction of the rosy apple aphid, *Dysaphis plantaginea* (Passerini) (Hemiptera: Aphidae) (Van Tol et al. 2009, *supra*). Typically, closed dispensers do not release high enough amounts of the plant volatiles thus, keeping the are typically left partially opened. The dispenser is a simple and cheap existing design, known in the art, which enables a high release profile especially for larger quantities of plant volatiles. In exemplary embodiments, release rates of about 0.06 ml/day or 0.0024 ml/hour are achieved. In other exemplary embodiments release rates of between about 0.02 to 0.1 ml/day are achieved using this method. In still other exemplary embodiments a release rate range of attractant VOCs is between about 1 ul/hour to 240 ul/hour.

In another exemplary embodiment, a blend of attractant VOCs is contained in a release membrane made from, for example, polyethylene, polypropylene, polyvinylchloride, mylar, and acrylic as described in e.g., Leonhardt et al, Insect Pheromone Technology: Chemistry and Applications, ACS Symposium Series 190, 1982 and Kydonisus, Controlled Release Pesticides, ACS Symposium Series 53, 1977; which are herein incorporated by reference. In some exemplary embodiments, efficacy and composition of attractive VOC is affected by the nature of the crop or nursery stock and by the particular weevil species being attracted. Thus, in some exemplary embodiments different combinations of odors are attractive to the different weevil species.

### IV. Traps for Otiorhynchus weevils

In an exemplary embodiment, the invention provides an apparatus that includes a movable housing or device that can be strategically placed within an area needing treatment. The apparatus may include a source of at least one volatile organic compound (VOC) as disclosed herein contained within or in the proximity of the housing and a dispenser that is adapted to initiate the release e.g., controlled release over a certain time period, of the composition over an area in need of treatment.

Typically, *Otiorhynchus* weevils leave plants they feed on at night to seek a hiding place during the day. Thus, in an exemplary embodiment, a minimal trap for *Otiorhynchus* weevils is a hiding place e.g., a piece of cardboard, from which the *Otiorhynchus* weevils can escape if they so choose. Although the *Otiorhynchus* weevils can escape such a trap, close monitoring of traps permits the horticulturalist to capture hiding weevils before they leave their hiding places at night.

In other exemplary embodiments, the attractant compositions of the present invention are used in conjuction with a trap by dissolving attractant in a non-volatile and non- solvent, such as, for example, mineral oil, ethylene glycol or any solid organic or non-organic material. This slow-release product is then placed in a trap vial or that is placed in the canopy of the plant. *Otiorhynchus* weevils attracted to the solution, will enter the trap. In another exemplary embodiment, the trap is the attractant VOCs dissolved in a non-volatile solvent, and placed in a trap system that incorporates a fast acting insecticide or biological agent(s) that will kill attracted *Otiorhynchus* weevils that visit the trap. The attractant can also be formulated into a controlled release matrix that attracts *Otiorhynchus* weevils.

In some exemplary embodiments, the attractant VOCs or blends of attractant VOCs is/are microencapsulated, by methods known in the art (*see e.g*., Bakan, J. A. Microencapsulation Using Coacervation/Phase Separation Techniques. In Controlled Release Technology: Methods, Theory, and Application; Kydonieus, A. F., Ed.; CRC Press: Boca Raton, FL, 1980; pp 83-105; and Herbig, S. M, et al. (1987) Am. Chem. Soc. Div. Polym. Chem. Prepr. 1987, 28, 92-9, each of which are incorporated herein by reference). However, any suitable method known in the art for dispersal/dispensation of volatile organic compounds disclosed herein for luring and/or trapping may be used *e.g.,* CheckMate® Puffer by Suterra.

In some exemplary embodiments, the attractant VOCs or blends of attractant VOCs are used with a trap designed for use in attracting and/or trapping *Otiorhynchus* weevils (a "weevil trap"). A typical weevil trap comprises a container which may be of variable form and size made of variable material. In an exemplary embodiment, a Wiffle® ball is used to construct the container part of the trap. Thus the trap comprises openings large enough for the weevils to enter. In the interior of the trap is a "ruffle" which is serves as a hiding place for weevils once they enter the trap. Typically, a "weevil trap" trap is placed in a tree or other plant wherein it is desired that weevils are to be trapped/captured/monitored etc. In some exemplary embodiments twigs and/or branches of the tree/plant are woven through holes in the trap. To trap weevils, the weevil trap is placed above and not on the ground. In some exemplary embodiments, the trap is place in a plant or in a plant canopy. Thus, in an exemplary embodiment, the hiding place takes the form of ruffle in a container with holes placed in the tree. In other exemplary embodiments, the trap is simply raised off the ground, by any suitable means.

### V. Exemplary Uses of Invention

In exemplary embodiments the invention is used as for monitoring, control, and/or detection of *Otiorhynchus* weevils. In one exemplary embodiment, the trap is deployed to tabulate the catch to determine size and location of *Otiorhynchus* weevil infestation. Economic and effective use of appropriate pest management systems can then be determined. In other exemplary embodiments, trapping the *Otiorhynchus* weevils serves as a control method.

In some exemplary embodiments attractant VOCs as disclosed herein are used in combination with insecticide application or other control measures. Thus, in one exemplary embodiment, the invention is used to attract *Otiorhynchus* weevils and to induce them to enter a trap or location where they contact an effective amount of toxicant to achieve control. An effective amount of the toxicant is an amount that is lethal for an exposed *Otiorhynchus* weevil or at least sublethal but sufficient to incapacitate the *Otiorhynchus* weevil with regard to future oviposition activity. Exemplary of the wide variety of toxicants which may be used with the invention are, e.g., methomyl, malathion, dichlorvos, acephate, indoxacarb and biological agents including but not limited to fungi, nematodes and bacteriar a combination of two or more of the above.

In some exemplary embodiments, the attractant VOC(s) disclosed herein are used in conjunction with a "weevil trap" (see e.g., Example 3 hereinbelow). In some exemplary embodiments, the attractant VOC(s) disclosed herein are used in conjunction with a "weevil trap" and an effective amount of toxicant to achieve control.

In still other exemplary embodiments, control of *Otiorhynchus* weevils can be achieved by using the invention to detect the location and boundaries of localized *Otiorhynchus* weevil infestations and employ in the area chemosterilants, bioregulator agents, parasites, predators or other biological control agents..

The following examples are offered to illustrate the invention.

### EXAMPLES

### Example 1:

The following example illustrates formulation of volatile organic compounds suitable for attraction of *Otiorhynchus* weevils.

### METHODS AND MATERIALS FOR EXAMPLE 1

Volatiles were collected from a bio-active extract of *Euonymus fortunei* 'Dart's Blanket' in paraffin oil and from cuttings of mechanically damaged and *O*. *sulcatus-*damaged *E. fortunei* 'Dart's Blanket'. The antennal response to these plant odours by *O*. *sulcatus* were measured by gas chromatography coupled with electroantennogram detection (GC-EAD). Compounds giving an electrophysiological response were tentatively identified by gas chromatography coupled with mass-spectrometry (GC-MS). When the tentatively identified compound showed similar Kováts indices on our chromatographic system as the purchased synthetic reference compound, it was considered to be a positive identification. A selection of plant volatiles that gave an electrophysiological reaction on the antenna were tested as single compounds and in several mixtures of two or more compounds on bioactivity using the olfactometer developed specifically to study vine weevil behaviour (*see e.g.,* Van Tol et al., (2002) Physiological Entomology 27, 213-222). A small selection of compounds bioactive in the lab was tested under field conditions in strawberry.

### Headspace collection

A filtered 10-ml extract of *E.fortunei* 'Dart's Blanket' in paraffin oil (Merck, Uvasol) and four *O*. *sulcatus-damaged* and four mechanically damaged 20-cm long cuttings of field-grown *E.fortunei* 'Dart's Blanket' were used for headspace collection. The extract was prepared by crushing 30 g of fresh leaves from the top 15cm part of stems with 50 ml of paraffin oil on ice. The extract was crushed for approximately 20 min followed by immediately filtering through Whatman No. 90 (diameter 15cm) paper filter. The filtered *Euonymus* extract was stored cool until use. Cuttings of *Euonymus* were mechanically damaged with a scissor by four incisions per leaf three hours prior to headspace collection.

The *O*. *sulcatus*-damaged *Euonymus* cuttings were prepared by offering 10 vine weevils 16 hrs prior to headspace collecting the cuttings. Weevils were removed and cuttings washed with deionized water prior to placing in a fresh 100 ml flask with water for headspace collection. For three days the *Euonymus* cuttings or a daily refreshed 10-ml paraffin oil extract of *Euonymus* in an open Petri-dish were placed under a glass bell jar (5 1) in a growth chamber at 20°C and under long day (L:D= 16:8 hr) light conditions. Air was purified by passage through a charcoal filter and drawn at 0.2 1 min ⁻¹ through the jar. Volatiles were entrained for a total of 69 hrs. For the collecting of volatiles, Gerstel thermodesorption tubes, filled with 80 mg Tenax TA 20/35 mesh (Grace Alltech), were used. Before use, these tubes were cleaned by rinsing them with 10 ml hexane and, subsequently, flushing them for one hour at 280°C with 20 ml min purified nitrogen. Each Tenax tube was refreshed after approximately 8 hrs. The volatiles trapped on the Tenax were washed off with 15 ml hexane. The Tenax washings for each treatment were pooled and concentrated under argon to one extract (∼200 µl) prior to GC-EAD and GC-MS testing (see e.g., Marco D'Alessandro and Ted C. J. Turlings (2006) Analyst 131:24-32; R.W.H.M. van Tol et al. (2009) Bulletin of Entomological Research 99: 593-602).

### Coupled gas chromatography electroantennographic detection (GC-EAD)

GC-EAD measurements were carried out using an Interscience Trace GC-2000 (Interscience, Breda, The Netherlands) equipped with a cold on-column injector. The gas chromatograph was equipped with a Grace-Alltech 30 m EC-5 fused silica column, 0.25-mm ID and 0.25-mm film thickness. Conditions were: carrier gas, helium (constant flow 1.7 ml min ⁻¹); temperature programming, 80°C (0.8 min hold) to 260°C (10 min hold) at 25°C min ⁻¹; detector temperature, 250°C; the transfer line between the GC and the EAD (Syntech Laboratories, Hilversum, The Netherlands) followed the oven temperature. Over the antenna, a flow of purified, humidified air was maintained at a flow rate of 80 cm sec ⁻¹. The sample was equally split between a flame ionization detector (FID) and the EAG detector. Antennae were separated from the weevil heads and mounted between two glass electrodes filled with a ringer solution (6.4mM KCl, 12mM MgCl2.6H2O, 9.6mM KOH, 12mM NaCl, 20mM KH2PO4, 1mM CaCl2 and 354mM glucose in deionized water). Antennal preparation and EAG recording were performed according to the procedure described by Visser & Piron (1995) and Van Tol et al. (2002b). The EAG recorder plus peripheral equipment were manufactured by Syntech Laboratories. Approximately five antennal preparations with limited background noise for each treatment showing responses to several compounds in the extract were used for comparison. Only EAG responses that were present in all preparations at the same retention time (Rt) were identified as an EAG positive response to a compound in the extract.

### Gas Chromatography-Mass Spectrometry (GC-MS)

GC-EAD active compounds were identified by mass spectrometry by injecting the same extracts used for GC-EAD on a GC-MS system under comparable conditions. GC-MS analyses were carried out on a Hewlett Packard 5973 mass selective detector (70 eV) coupled to a Hewlett Packard 6890 gas chromatograph equipped with a split/splitless injector. The gas chromatograph was equipped with an Alltech 30 m AT-5 fused silica column, 0.25 mm ID and 0.25 µm film thickness run in constant flow mode (1.3 ml/min Helium). Temperature programming: 50°C (2 min hold) to 300°C (8 min hold) at 5°C/min (AT-5 column); transfer line temperature, 300°C; injector temperature, 230°C. One µl of concentrated headspace volatiles were injected manually into the GC-MS system for analysis. Injections were done in splitless mode only (1 µl).

### Compounds and treatments

A selection of single plant compounds (A = (Z)-2-pentenol and B = (E)-2-hexenol) and combinations of plant compounds (C = A+B in ratio 1:1, D = A+methyl eugenol in ratio 1:1, and E = A+B+methyl eugenol in ratio 1:1:1) were tested in a strawberry field (*Fragaria* × *ananassa* 'Tillamook') for attraction of *O*. *sulcatus.* Choice of compounds and mixtures were based on identification of EAD-active compounds from headspace of weevil-damaged *E. fortunei* 'Dart's Blanket' plants and paraffin oil extract of the same *Euonymus* plant species combined with bioassay results with the EAD-active compounds alone and in several mixtures (Van Tol, unpublished). The compound (Z)-2-pentenol (purity 95%) was obtained from Bedoukian (Danbury, CT, USA), (*E*)-2-hexenol (purity >96%) from Acros (Geel, Belgium) and methyl eugenol (purity >98%) from Sigma-Aldrich (St. Louis, Missouri, USA). All chemicals were used without further purification. Single compounds and mixtures were introduced in dispensers prior to use and sealed. In each dispenser 0.4 ml of each compound was present.

### Dispensers

Plant volatile dispensers were made of 1.5 ml LDPE Pasteur pipettes (Labo Scientific, Ede, the Netherlands). The compounds to be tested were introduced into the pipette, the tip of which was then sealed by heat. Prior to use, the tip of the pipette was cut off at 1 cm above the reservoir portion. The open tip of the dispenser had an internal diameter of 3.5 mm. This type of 'high release' pheromone/kairomone dispenser are developed and used by Pherobank (www.pherobank.com) for several years for the attraction of *Phyllopertha horticola* L. (Coleoptera: Scarabaeidae) and has also been successfully tested for attraction of the rosy apple aphid, *Dysaphis plantaginea* (Passerini) (Hemiptera: Aphidae) (Van Tol et al, 2009). Closed dispensers do not release high enough amounts of the plant volatiles through the polyethylene to attract these beetles and aphids compared to partially opened vials. The dispenser is a simple and cheap existing design which enables a high release profile especially for larger quantities of plant volatiles. The plant volatiles were present as pure commercial compounds single or mixed in one dispenser according to the compound composition.

### Field experiment

Experiments were performed on a commercial strawberry field in Oregon (USA). Five different treatments (A, B, C, D and E) and one control were tested in the field. The control consisted of an empty dispenser. Dispensers with odors were placed in the top part of a boll weevil trap (Great Lakes IPM, Inc., Vestaburg, MI). Each trap contained one odor-filled or control (empty) dispenser located in the top capture assembly. Boll weevil traps were placed in the rows between the strawberry plants. Distance between each treatment was 10 meters. Each treatment of trap-odor was coupled with a trap-control treatment at a distance of 10 meters from each other and replicated four times for each field trial. Coupled odor-control set-up was performed to minimize effects of possible uneven weevil distribution throughout the fields. Dispensers were refreshed once a week. Traps and dispensers were placed in the fields and monitored for weevils' presence between May and August 2009. First weevils emerging from soil were found in the first week of June. Traps and plants surrounding each trap-dispenser combination were monitored for weevils weekly. Plants within the treatment row up to 60 cm distance from the dispenser in either direction were checked for weevil presence. Weevils found were removed from the field.

### Statistics

The field tests were set-up as block designs where the blocks consisted of four plots. Each plot was divided into two subplots where one subplot was the trap-odor treatment and the other subplot the trap-control treatment. The four subplots within a block were treated with the same odor. Each block contained therefore four identical odor traps coupled with four control traps. This set-up allows comparison of each odor with the control but not comparison between the different odors tested.

The total number of weevils for each weevil species per trap were analyzed using GLM (Generalized Linear Model) with logarithmic link, Poisson distribution and not fixed dispersion using the 12^{th} version of the statistical package GenStat (Payne et al., 2009). The fixed part of the model consists of the additive effects of block/plot and odor (of which the control was an extra level). After the analysis, paired comparisons were performed on the transformed scale data with approximate t-tests between the odors and the control. Thereafter, estimates of the means of the weevils per trap are back transformed to the original scale with approximate standard errors.

### RESULTS FOR EXAMPLE 1

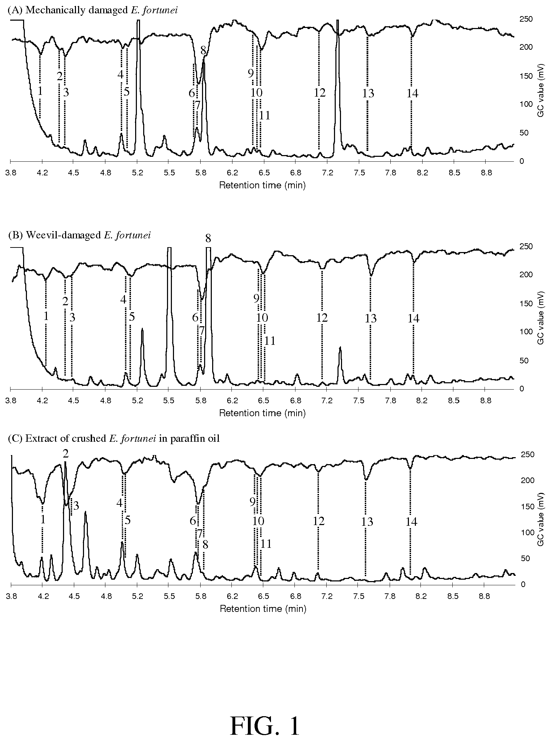

The headspace of mechanically damaged and weevil-damaged *E. fortunei* 'Dart's Blanket' plants and the leaf extract in paraffin oil showed a different pattern in release of compounds (FIG. 1). EAD-active compounds were present in all three headspace extracts and only differences in strength of antennal responses were found. Without being bound by theory it is believed that the differences in strength are related to the amount of the specific compounds present in the headspace of the different treatments. The amounts of compounds and strength of the antennal responses were not quantified in this research. The results show that of the 14 EAD-active volatiles (*Z*)-2-pentenol, (*Z*)-3-hexenol and (*E*)-2-hexenol were present in larger quantities in the headspace of the *Euonymus* extract in paraffin oil than in the headspace of mechanically and weevil-damaged *Euonymus* leading to clearly stronger antennal responses for these compounds in the extract compared to the plant headspaces. High release of EAD-positive DMNT in weevil-damaged *Euonymus* compared to the mechanically damaged plant and paraffin oil extract indicate that this compound is produced and released by the plants in increased amounts as a response to weevil damage. There was, however, no stronger antennal response to this compound in the weevil-damaged plants compared to the other treatments.

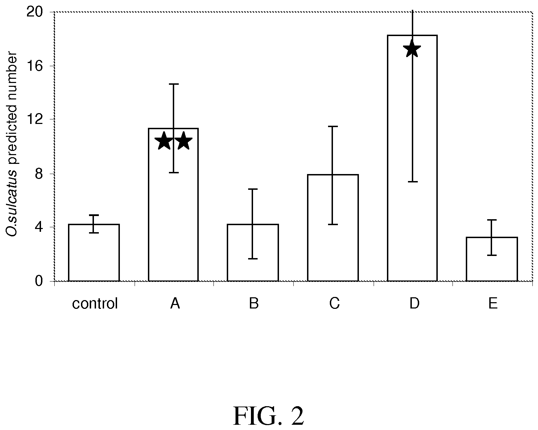

Results presented in FIG. 2 show the number of weevils per replicate for each treatment after statistical analysis of the total number of each weevil species caught during the whole season. Except for an occasional weevil caught in the boll weevil trap all weevils were found in the plants surrounding the odor source.

Treatment A (11.3, s.e. = 3.3, *p* = 0.01) and D (18.2, s.e. = 10.9, *p* = 0.03) caught significant more *O*. *sulcatus* than the control (4.3, s.e. = 0.7). The treatments B (4.3, s.e. = 2.6, *p* = 1.0), C (7.9, s.e. = 3.6, *p* = 0.22) and E (3.3, s.e. = 1.3, *p* = 0.56) were not significantly different from the control.

### Discussion

This is the first report of a successful attraction of weevils in the genus *Otiorhynchus* to a synthetic kairomone comprising plant volatiles. Three compounds from the spindle tree *Euonymus fortunei* 'Dart's Blanket', attractive for *O*. *sulcatus* (Van Tol et al., 2002a), and sensed by their antenna play a role in attraction. Of these compounds (*Z*)-2-pentenol alone and in combination with methyl eugenol is attractive for *O*. *sulcatus* in strawberry. For two other weevil species in the same genus (*O. ovatus* and *O*. *rugosostriatus*), present in the strawberry test field, there was attraction to a mixture of (*Z*)-2-pentenol and (*E*)-2-hexenol but not to the *O*. *sulcatus* attractive treatments.

Many weevils in the genus *Otiorhynchus* are polyphagous and reproduce parthenogenetically. Thus, without being bound by theory it is believed that unless these weevil species also produce an aggregation pheromone it is likely that plant odors play a role in host-plant finding and/or aggregation (e.g. feeding-induced release of plant compounds attracting conspecifics). Indeed, EAD profile of *O*. *sulcatus* (Van Tol et al, 2002b) showed strong antennal responses to typical Green Leaf Volatiles (GLVs) with strongest responses to the C6 alcohols but not the C6 aldehydes, acetates or ketones.

The headspace of a preferred *O*. *sulcatus*-damaged host-plant (*E. fortunei*) and an attractive host-plant extract (Van Tol, unpublished) tested on weevils' antennal response via GC-EAD coupled with GC-MS analysis revealed responses to two C6 alcohols present in the headspace of the plants, namely (*Z*)-3-hexenol and (*E*)-2-hexenol and also to a C5 alcohol, (*Z*)-2-pentenol. In laboratory and field tests only (*Z*)-2-pentenol was attractive to *O*. *sulcatus* next to methyl eugenol. Pentenols are compounds found in several plant species released in substantial amounts when exposed to pathogen attack (Heiden et al., 1999) and after freeze-thaw wounding of plants (Fall et al., 2001).

To our knowledge no behavioral responses of any insect species to pentenols has been described prior to this disclosure. The other compound, attractive in conjunction with (Z)-2-pentenol for *O*. *sulcatus* - methyl eugenol - occurs naturally in plants from over 200 species in 32 families (Tan, 2000).

Thus, in exemplary embodiments, odor composition is optimized for use in trap devices for monitoring in the field. *Otiorhynchus* weevils are night active feeders and are attracted to the host plants near the odor source at night while the traps tested are designed to act as daytime hiding locations and are not attractive for the weevils to enter during feeding. In an exemplary embodiment, new trap devices are constructed for capture efficacy.

### Example 2:

The following example illustrates formulation a synergistic effect of light on attraction of *Otiorhynchus* weevils exposed attractant volatile organic compounds.

A solar powered LED light (X watts) was positioned 30 cm above the ground in close proximity of the plant containing an *Otiorhynchus* sp. traps (described hereinbelow in Example 3). The light contained a sensor that powered the LED during the overnight hours. The proportion of *Otiorhynchus sulcatus* adults in the traps near a light source the following morning were compared to those traps absent of light. Results are shown in Table 1 below.

**Table 1. Proportion of Otiorhynchus weevils recaptured from two example traps tested with supplemental overnight (0.1-1.1 lux) in comparison to the same trap absent of light in 2010.**

| Experimental date | Trap A | Trap B |
|---|---|---|
| 7/8 June | * | .81 |
| 9/10 June | * | .65 |
| 14/15 June | .18 | 0.0 |
| 17/18 June | .42 | .60 |
| 24/25 June | .56 | 1.0 |

| | | |
|---|---|---|
| *No supplemental light treatment performed | | |

As can be seen in the Table, a larger proportion of trapped weevils were trapped with supplemental overnight light, than were trapped without supplemental overnight light. For example, 7/8 June data Trap B. Here the data shows that all else being the same, 81% of the trapped weevils were trapped with the application of overnight light.

### Example 3:

The following example illustrates a trap designed for use in attracting and/or trapping *Otiorhynchus* weevils.

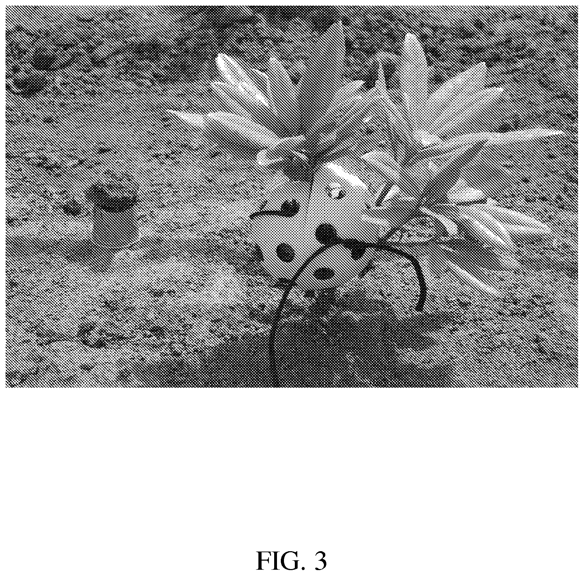

In an exemplary embodiment a trap designed specifically for trapping *Otiorhynchus* weevils is used. An Exemplary *Otiorhynchus* weevil trap (or "weevil trap") is shown in FIG. 3.

In general, a trap designed for use in attracting and/or trapping *Otiorhynchus* weevils comprises a container which may be of variable form and size made of variable material. In an exemplary embodiment, a Wiffle® ball is used to construct the container part of the trap. Thus the trap comprises openings where weevils can enter. The number and size of openings are not important except in-so-far as the openings must be large enough for the weevils to enter.

In the interior of the trap is a "ruffle" which is serves as a hiding place for weevils once they enter the trap. The "ruffle" is, as generally understood a gathered or goffered fluted frill of lace or cloth or paper or any convenient material. Exemplary "ruffle" material includes, but is not limited to cotton ruffles from the border of a garment.

Typically, the trap is placed in a tree or other plant wherein it is desired that weevils are to be trapped/captured/monitored etc. In some exemplary embodiments twigs and/or branches of the tree/plant are woven through holes in the trap.

To trap weevils, the weevil trap is placed above and not on the ground. In some exemplary embodiments, the trap is place in a plant or in a plant canopy. In other exemplary embodiments, the trap is simply raised off the ground, by any suitable means available to a person having ordinary skill in the art.

In some exemplary embodiments, the trap is used in combination with light as described in Example 2. In other exemplary embodiments, the trap is used with various sources of light and attractants VOC(s). In still other exemplary embodiments the trap is used with attractant VOC(s) and in still other exemplary embodiments, the trap is used alone without attractant VO(s) or light. In some exemplary embodiments, the trap is used in combination with any attractive and/or killing bait and thus in some exemplary embodiments is a "lure and kill" device.

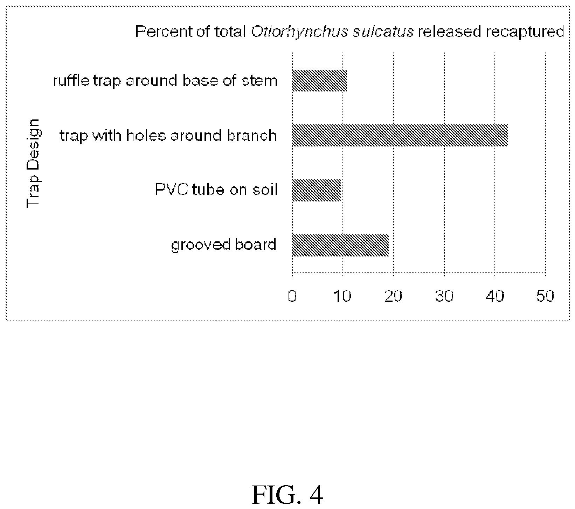

Field studies were performed in 2010 to test the capture efficacy of several *Otiorhynchus* sp. trap designs. Traps tested included a ruffle of cotton secured around the base of a plant stem, a weevil trap as disclosed hereinabove (see e.g., FIG. 3) secured in the canopy of the plant, a PVC tube with four opening on the soil surface, and a standard grooved board. The experiment was replicated eight times over a period of four days. For each replicate, a single *Rhododendron* sp. plant was enclosed in 1 m³ cage and 20 weevils (starved for 24 hrs) were released in each cage in late afternoon. The following morning, the cages were carefully inspected and the number of weevils captured in each trap design tabulated. The data is shown in FIG. 4.

The data shown in FIG. 4 indicate that the "weevil trap" recaptured more than twice as many of the released weevils as any other trapping device tested.

## Claims

1. Use of a composition for attracting Otiorhynchus weevils, wherein said composition consists of a volatile organic compound of at least 85 % purity selected from the group consisting of (X)-Y-pentenol, methyl eugenol, methyl iso-eugenol, and a combination of said members, wherein X is E or Z, and Y is 1 or 2.

2. The use of claim 1, wherein the Otiorhynchus weevils are Otiorhynchus sulcatus

3. The use of claim 2, wherein said composition consists of (Z)-2-pentenol of at least 85 % of purity.

4. The use of claim 2, wherein said composition consists of the combination (Z)-2-pentenol and methyl eugenol of at least 85 % of purity.

5. The use of claim 1, wherein the Otiorhynchus weevils are members selected from the group consisting of Otiorhynchus ovatus and Otiorhynchus rugosostriatus, and the composition consists of (Z)-2-pentenol of at least 85 % purity.

6. A trap for capturing Otiorhynchus weevils,
- wherein the trap comprises a container that has an interior and an exterior, wherein the container comprises openings in the exterior that are large enough for the weevils to pass through and thereby enter the interior; and wherein the interior contains a "ruffle" which serves as a hiding place for weevils that enter the trap ; and
- wherein the trap is baited with an attractant composition consisting of a volatile organic compound of at least 85 % purity that are members selected from the group consisting of (X)-Y-pentenol, methyl eugenol, methyl iso-eugenol and a combination of such members, wherein X is E or Z, and Y is 1 or 2.

7. The trap of claim 6, wherein the trap is placed in a tree or other plant wherein it is desired that weevils are to be trapped/captured/monitored.

8. The trap of claim 6, wherein the trap is baited with a composition consisting of (Z)-2-pentenol of at least 85 % purity.

9. The trap of claim 6, wherein the trap is baited with a composition consisting of (Z)-2-pentenol and methyl eugenol of at least 85 % purity.

10. The trap of claim 6, wherein the trap is baited with a composition consisting of a volatile organic compound of at least 85 % of purity that are members selected from the group consisting of (Z)-2-pentenol, methyl eugenol and a combination of such members.

11. The trap of claim 6, wherein the trap further contains a bait laced with synthetic insecticide or pathogens.

12. The trap of claim 6, wherein the pathogens are members selected from the group consisting of bacteria, fungi, nematodes and microspora.

13. The trap of claim 6, wherein the trap is used in conjunction with light at an intensity of between about 0.1-1.1 lux for the duration of overnight hours.

## Patentansprüche

1. Verwendung einer Zubereitung zum Anlocken von *Otiorhynchus-*Rüsselkäfern, wobei die Zubereitung besteht aus einer flüchtigen organischen Verbindung von wenigstens 85 % Reinheit, die gewählt ist aus der Gruppe, die besteht aus (X)-Y-Pentenol, Methyleugenol, Methylisoeugenol und einer Kombination der Verbindungen, wobei X für E oder Z steht und Y 1 oder 2 ist.

2. Verwendung nach Anspruch 1, wobei die *Otiorhynchus*-Rüsselkäfer *Otiorhynchus sulcatus* sind.

3. Verwendung nach Anspruch 2, wobei die Zubereitung besteht aus (Z)-2-Pentenol von wenigstens 85 %-iger Reinheit.

4. Verwendung nach Anspruch 2, wobei die Zubereitung besteht aus der Kombination (Z)-2-Pentenol und Methyleugenol von wenigstens 85 %-iger Reinheit.

5. Verwendung nach Anspruch 1, wobei die *Otiorhynchus*-Rüsselkäfer sind, die gewählt sind aus der Gruppe, die besteht aus *Otiorhynchus ovatus* und *Otiorhynchus rugosostriatus,* und wobei die Zubereitung besteht aus (Z)-2-Pentenol von wenigstens 85 %-iger Reinheit.

6. Falle zum Fangen von *Otiorhynchus*-Rüsselkäfern,
- wobei die Falle einen Behälter umfasst, der ein Inneres und ein Äußeres aufweist, wobei der Behälter Öffnungen in dem Äußeren umfasst, die groß genug sind dafür, dass die Rüsselkäfer hindurchpassen und dadurch in das Innere hineinkriechen, und wobei das Innere ein "Kräuselmaterial" enthält, das als Versteck für Rüsselkäfer dient, die in die Falle hineinkriechen; und
- wobei die Falle beködert ist mit einer Köder-Zubereitung, die besteht aus einer flüchtigen organischen Verbindung von wenigstens 85 % Reinheit, die Verbindungen sind, die gewählt sind aus der Gruppe, die besteht aus (X)-Y-Pentenol, Methyleugenol, Methylisoeugenol und einer Kombination der Verbindungen, wobei X für E oder Z steht und Y 1 oder 2 ist.

7. Falle nach Anspruch 6, wobei die Falle platziert ist in einem Baum oder einer anderen Pflanze, von denen es erwünscht ist, dass die Rüsselkäfer darin gefangen / eingefangen / überwacht werden.

8. Falle nach Anspruch 6, wobei die Falle beködert ist mit einer Zubereitung, die besteht aus (Z)-2-Pentenol von wenigstens 85 %-iger Reinheit.

9. Falle nach Anspruch 6, wobei die Falle beködert ist mit einer Zubereitung, die besteht aus (Z)-2-Pentenol und Methyleugenol von wenigstens 85 %-iger Reinheit.

10. Falle nach Anspruch 6, wobei die Falle beködert ist mit einer Zubereitung, die besteht aus einer flüchtigen organischen Verbindung von wenigstens 85 % Reinheit, die Verbindungen sind, die gewählt sind aus der Gruppe, die besteht aus (Z)-2-Pentenol, Methyleugenol und einer Kombination solcher Verbindungen.

11. Falle nach Anspruch 6, wobei die Falle weiter einen Köder enthält, der versetzt ist mit synthetischem Insektizid oder Pathogenen.

12. Falle nach Anspruch 6, wobei die Pathogene Elemente sind, die gewählt sind aus der Gruppe, die besteht aus Bakterien, Pilzen, Nematoden und Mikrosporen.

13. Falle nach Anspruch 6, wobei die Falle verwendet wird in Verbindung mit Licht bei einer Intensität zwischen etwa 0,1 bis 1,1 lux für die Dauer der Nachtstunden.

## Revendications

1. Utilisation d'une composition pour attirer les charançons Otiorhynchus, dans laquelle ladite composition consiste en un composé organique volatil pur à au moins 85% sélectionné parmi un groupe consistant en du (X)-Y-penténol, du méthyleugénol, du méthyl isoeugénol et en une combinaison desdits éléments, dans laquelle le X est E ou Z, et Y est 1 ou 2.

2. Utilisation selon la revendication 1, dans laquelle les charançons Otiorhynchus sont des Otiorhynchus sulcatus.

3. Utilisation selon la revendication 2, dans laquelle ladite composition consiste en du (Z)-2-penténol pur à au moins 85%.

4. Utilisation selon la revendication 2, dans laquelle ladite composition consiste en une combinaison de (Z)-2-penténol et de méthyleugénol purs à au moins 85%.

5. Utilisation selon la revendication 1, dans laquelle les charançons Otiorhynchus ont été sélectionnés parmi un groupe d'Otiorhynchus ovatus et d'Otiorhynchus rugosostriatus et la composition consiste en du (Z)-2-penténol pur à au moins 85%.

6. Piège pour capturer les charançons Otiorhynchus,
- dans lequel le piège comprend un contenant avec un intérieur et un extérieur, dans lequel le contenant comprend des ouvertures dans l'extérieur suffisamment grandes pour laisser passer les charançons et ainsi les laisser pénétrer à l'intérieur, et dans lequel l'intérieur contient une « fronce » qui permet aux charançons qui sont entrés dans le piège de s'y cacher ; et
- dans lequel le piège est garni d'une composition servant d'appât consistant en un composé organique volatil pur à au moins 85% dont les éléments sont sélectionnés parmi un groupe consistant en du (X)-Y-penténol, du méthyleugénol, du méthyl isoeugénol, et en une combinaison de ces éléments, dans laquelle X est E ou Z, et Y est 1 ou 2.

7. Piège selon la revendication 6, dans lequel le piège est placé dans un arbre ou une autre plante dans lequel on souhaite piéger/capturer/surveiller les charançons.

8. Piège selon la revendication 6, dans lequel le piège est garni d'une composition servant d'appât consistant en du (Z)-2-penténol pur à au moins 85%.

9. Piège selon la revendication 6, dans lequel le piège est garni d'une composition servant d'appât consistant en du (Z)-2-penténol et du méthyleugénol purs à au moins 85%.

10. Piège selon la revendication 6, dans lequel le piège est garni d'une composition servant d'appât consistant en un composé organique volatil pur à au moins 85% dont les éléments sont sélectionnés parmi un groupe consistant en du (Z)-2-penténol, du méthyleugénol et en une combinaison de ces éléments.

11. Piège selon la revendication 6, dans lequel le piège comprend en outre un appât additionné d'insecticide de synthèse ou d'agents pathogènes.

12. Piège selon la revendication 6, dans lequel les agents pathogènes sont des éléments sélectionnés parmi le groupe constitué par les bactéries, les champignons, les nématodes et les microspores.

13. Piège selon la revendication 6, dans lequel le piège est utilisé conjointement avec de la lumière d'une intensité comprise entre environ 0,1 et 1,1 lux pendant la durée de la nuit.
